# EUROPÄISCHE PATENTSCHRIFT

(11) **EP 2 198 267 B1**
(45) Veröffentlichungstag und Bekanntmachung des Hinweises auf die Patenterteilung: **06.11.2013**
(21) Anmeldenummer: 08838699.0
(22) Anmeldetag: 20.09.2008
(51) Int. Cl.: G01N 19/02, G01N 33/42

(54) **PRÜFGERÄT ZUR MESSUNG UND PROGNOSE DER GRIFFIGKEIT VON FAHRBAHNDECKSCHICHTEN UND VERFAHREN**
TEST DEVICE FOR MEASURING AND FORECASTING THE GRIP OF ROAD SURFACE LAYERS AND METHOD
APPAREIL DE CONTRÔLE POUR MESURER ET PRONOSTIQUER L'ADHÉRENCE D'UN REVÊTEMENT DE CHAUSSÉE ET PROCÉDÉ ASSOCIÉ

(30) Priorität: 19.10.2007 DE 102007050137
(43) Veröffentlichungstag der Anmeldung: 23.06.2010
(73) Patentinhaber: BEAB Brandenburg H. Engineering & Anlagenbau GmbH, 14770 Brandenburg a.d. Havel (DE)
(72) Erfinder: FRANKE, Dietmar, 14797 Kloster Lehnin (DE); BUNTINS, Lutz, 14770 Brandenburg a. d. Havel (DE)
(74) Vertreter: Schulte, Jörg
(86) Internationale Anmeldenummer: PCT/DE2008/001569
(87) Internationale Veröffentlichungsnummer: WO 2009/049582

(56) Entgegenhaltungen:
- EP-A- 0 193 521
- DE-A1- 3 409 040
- DE-A1-102004 044 095
- GB-A- 2 419 962
- JP-A- 2004 085 317
- US-A- 4 050 290
- US-A1- 2004 187 556
- US-B1- 7 000 451

## Beschreibung

Die Erfindung betrifft ein Prüfgerät zur Messung der Griffigkeit von Fahrbahndeckschichten mit einem in einem bestimmten Winkel entlang einer Bahn über einen Antrieb gezogenen, schräg laufenden Laufrad und einer die notwendigen Messdaten ermittelnden Messeinrichtung, wobei das Laufrad (5) rotierend und zwei beabstandete, schräg laufende Messräder (9, 11) mit Messreifen (10, 12) aufweisend ausgebildet ist, dass und wobei die Messeinrichtung (6) den Messrädern (9, 11) zugeordnet und die Seitenkraft und die Radlast oder Normalkraft ermittelnd ausgeführt ist. Außerdem betrifft die Erfindung ein Verfahren zur Messung der Griffigkeit von Fahrbahnoberflächen von Straßen und Wegen, wobei über ein bei etwa 10 - 20° gegenüber der Geraden schräg gestelltes Laufrad neben anderen notwendigen Messdaten die Kraft ermittelt wird, mit der das Laufrad versucht sich in die Fahrtrichtung zu verschwenken, wobei das Laufrad aus zwei im Abstand angeordneten Messrädern mit Messreifen besteht, und wobei die Seitenkraft und die Radlast kontinuierlich und stationär im Labor oder Feld gemessen und die gesamten ermittelten Messdaten ausgewertet werden.

Um den Straßenzustand einer Fahrbahndeckschicht auf Griffigkeit oder noch vorhandene Griffigkeit zu prüfen gibt es verschiedene maschinelle Verfahren. Dabei wird als Griffigkeit der Fahrbahn die Reibungskraft bezeichnet, die aufgewendet wird, um den Kraftschluss zwischen einem Fahrzeugreifen und der Fahrbahnoberfläche zu gewährleisten. Die Griffigkeit bestimmt somit als Oberflächeneigenschaft den Gebrauchswert einer Straße. Im Allgemeinen stellt die Griffigkeit einer Straße eine Reibungskraft dar, die der Relativbewegung zwischen den sich berührenden Reifen und der Fahrbahn entgegenwirkt. Schon in den 30er Jahren des vergangenen Jahrhunderts sind Messsysteme entwickelt worden, um die Griffigkeit von Straßenbelegen überprüfen zu können. Um die Griffigkeit einer solchen Straßenoberfläche zu bewerten, müssen die Kräfte gemessen werden, die zwischen Reifen und Fahrbahn übertragen werden können, bevor ein gefährliches Gleiten eintritt. Praktisch ist es schwierig, diesen Zustand der höchsten Ausnutzung der Griffigkeit messtechnisch zu erfassen. In der straßenbaulichen Praxis werden Kennwerte für die Griffigkeit benötigt, die messtechnisch möglichst einfach zu gewinnen sind. Dabei ist der Straßenbau an Messmethoden interessiert, mit der die Griffigkeitswerte über die Gesamtlänge der Straße kontinuierlich aufgenommen werden können, die jedoch auch das Messen in Kurven, Kreuzungsbereichen und an anderen besonders belasteten Streckenabschnitten erlauben. Die Griffigkeit von Fahrbahnoberflächen wird üblicherweise mit Fahrzeugen gemessen, die mit Messrädern oder Messanhängern ausgestattet sind. Als Messradvarianten sind das blockierte Rad, das unter kontrolliertem Schlupf abrollende Rad und das schräg gestellte Rad verbreitet. Entsprechende Versuchsgeräte sind in England, Frankreich, Holland und Deutschland gebaut und eingesetzt worden. In Deutschland wurde der Stuttgarter Reibungsmesser (SRM) von 1955 bis 1999 als Standardgerät eingesetzt. Da mit dem blockierten Rad (SRM) wegen des zu hohen Verschleißes an der Bremse der Fahrbahnbelag nicht kontinuierlich durchgemessen werden konnte, hat die Messmethode mit dem Schräglaufrad, die englische Sideway Force Coefficient Routine Investigation Machine (SCRIM), den Stuttgarter Reibungsmesser mit dem blockierten Rad abgelöst. Für die notwendigen Messungen gibt es auch Vorschriften. Neben dem dynamischen Messverfahren existieren auch Pendelmessgeräte, die stationär eingesetzt werden. Damit erhaltene Werte sind nicht immer genau, ebenso wie mit den anderen bekannten Geräten. Bei den Griffigkeitsmessungen kann das Niveau der Griffigkeit in ihrem aktuellen Zustand mit den bekannten Geräten eventuell noch festgestellt werden, eine Prognose der Griffigkeit über den Zeitpunkt des aktuellen Zustandes hinaus ist allerdings nicht möglich. Darüber hinaus sind einige Laborgeräte entwickelt worden, die es auch ermöglichen, bezüglich des zum Einsatz kommenden Straßenbelages eine Prognose mit abzugeben, die allerdings mit erheblichen Unsicherheiten belastet ist. Hierbei wird die Messung auf einem entsprechend lange polierten Prüfstück entsprechend wiederholt. Sowohl bei den allgemeinen Griffigkeitsmessungen, wie auch Prognoseverfahren ist von wesentlichem Nachteil, dass eine Vergleichbarkeit der Werte der unterschiedlichen Messmethoden untereinander nicht möglich ist. Zwar existieren Berechnungsmodelle für die Überführung der Werte untereinander, physikalisch sind die Werte allerdings nicht korrelierbar. Nachteilig ist außerdem das teilweise hohe Gewicht der Prüfgeräte, wobei das SCRIM-Gerät beispielsweise 380 kg wiegt und zwischen Vorder- und Hinterachse eines LKWs am Fahrzeugrahmen fest montiert werden muss.

Der Erfindung liegt daher die Aufgabe zu Grunde, ein transportables Prüfgerät für Straßendeckschichtüberprüfungen und ein entsprechendes Verfahren zu schaffen, über die reproduzierbare Messwerte und weiter eine Griffigkeitsprognose möglich wird.

GB2419962 offenbart ein Prüfgerät und ein Verfahren mit den Merkmalen des Oberbegriffs der Ansprüche 1 und 14.

JP2004085317 offenbart ein Prüfgerät zur Messung der Griffigkeit einer Oberfläche, wobei ein einziges Messrad über einen Rotationsantrieb entlang einer kreisförmigen Bahn gezogen ist.

Die Aufgabe wird gemäß der Erfindung dadurch gelöst, dass die Messräder mit einem als Rotationsantrieb ausgelegten Antrieb ausgerüstet sind und entlang einer kreisförmigen Bahn über den Antrieb gezogen sind.

Mit einem derart ausgebildeten Prüfgerät ist es zunächst einmal möglich, die Griffigkeit von Fahrbahndeckschichten direkt vor Ort oder aber auch im Labor zu überprüfen, wobei das Prüfgerät als solches problemlos transportiert werden kann. Da es an beliebiger Stelle eingesetzt werden kann, ist eine leichte Ermittlung der notwendigen Messwerte und damit eine Prüfung der Griffigkeit möglich. Die hier als Laufrad dienenden Messräder rotieren um eine feste Achse, sodass eine wesentlich genauere Aussage über den Zustand der Fahrbahndeckschicht abgegeben werden kann, als bisher überhaupt nur denkbar. Darüber hinaus kann die Messung beliebig häufig wiederholt werden und sei es, dass man das Prüfgerät geringfügig versetzt. Neben dem stationären Einsatz ist es auch möglich, ein solches Prüfgerät einem Fahrzeug zuzuordnen und dann diese Messung mehr oder weniger kontinuierlich durchzuführen, um so z. B. einen ersten Überblick zu erhalten. Sind dann auffällige "Negativwerte" ermittelt worden, so kann man problemlos die Messung an diesen Stellen wiederholen und feststellen, ob die ermittelten Messdaten zutreffend sind oder sich Fehler eingeschlichen haben. Die rotierenden Messräder versuchen wegen ihrer Schrägstellung genau wie beim Ziehen über eine lange gerade Bahn in die Fahrtrichtung zu gelangen. Die dabei zu ermittelnde Seitenkraft ist vom Schräglaufwinkel des Laufrades abhängig und erreicht ihren größten Wert je nach Art und Zustand der Straßenoberfläche bei etwa 10 - 20° und nimmt bei weiterer Vergrößerung des Winkels nicht mehr zu. Als weiterer erheblicher Vorteil ist bei dem beschriebenen Prüfgerät zu vermerken, dass aufgrund der wenigen Bauteile des Prüfgerätes und des auch vom Prinzip her einfachen Aufbaus ein Gerät zur Verfügung gestellt wird, das ohne großen Aufwand transportiert werden kann, sodass der Einsatzbereich praktisch unbegrenzt ist.

Nach einer zweckmäßigen Ausbildung der Erfindung ist vorgesehen, dass die Messeinrichtung einer der Felgen des Messreifens zugeordnet ist. Da hier die Messeinrichtung dort angeordnet werden kann, wo eine Beeinflussung oder gar Verformung des Reifens an sich nicht eintritt, können vorteilhaft genaue Werte der Seitenkraft oder des Seitenreibungsbeiwertes ermittelt werden.

Um die Genauigkeit der ermittelten Werte möglichst hoch ansetzen zu können, sieht die Erfindung vor, dass das rotierende Laufrad mit den Messrädern die durch den Rotationsantrieb und die durch die Messreifen entstehenden Schwingungen entkoppelnd im Gerätegehäuse gelagert ist. Eine entsprechende Lagerung oder Halterung ist ohne großen Aufwand zu erreichen, sodass eine Beeinflussung der ermittelten Messdaten hierdurch nicht mehr auftreten kann.

Die zum Einsatz kommende Messeinrichtung muss bestimmte Werte ermitteln, wobei der Seitenkraft- oder Seitenreibungsbeiwert µ_{RAG} als Quotient aus der auf das rotierende Laufrad wirkenden Normalkraft F_{N} und der reibungsbedingten Komponente F_{Ry} der gemessenen Seitenkraft F_{y'} ermittelbar ist, wobei die gemessene Seitenkraft F_{y'} sich aus der Summe der in Richtung y' wirkenden Komponenten der Zentrifugalkraft F_{Zy'} und Reibungskraft F_{Ry'} ergibt. Auf diese Weise erhält die erfindungsgemäße Messeinrichtung die notwendigen Messdaten, die es ihr ermöglichen, die gewünschten Beurteilungskriterien klar herauszustellen.

Die Messräder werden über den Rotationsantrieb auf 40, 60 und 80 km/h beschleunigt, sodass die Hauptbelastungsdaten für die Griffigkeit auch bei unterschiedlichen Geschwindigkeiten ermittelt werden können, was die Beurteilung insgesamt des jeweiligen Straßenbelages verbessert. Dabei kann es zweckmäßig sein, dass die einer der Felgen zugeordnete Messeinrichtung ein Kraftaufnahmesystem zur Bestimmung der Seitenkraft F_{y'} und der Radlast F_{N} aufweist. Ein solches Kraftaufnahmesystem kann in das Gesamtprüfgerät problemlos integriert werden.

Eine weitere Möglichkeit ist die, dass die Messeinrichtung zur Messung der auftretenden Seitenkraft und der Radlast bzw. der dadurch auftretenden Kräfte Druckaufnehmer aufweist. Diese, der Messeinrichtung zugeordneten Druckaufnehmer, bringen saubere und gut verwertbare Messdaten.

Die Messung der Seitenkraft kann gemäß der Erfindung auch oder zusätzlich ermittelt werden, wozu die Messeinrichtung zur Messung der Seitenkraft zusätzlich oder nur Dehnungsmessstreifen (DMS) aufweist, die zu einer Vollbrücke verschaltet sind. Das Signal der Seitenkraft ist vom Drehwinkel unabhängig, da die DMS so verschaltet werden, dass ein störender Einfluss von Lenk- und Sturzmomenten vollständig ausgeglichen werden kann.

Bei der Ausbildung des Prüfgeräts kann auch auf vorhandene weitere Messsysteme zurückgegriffen werden. So sieht die Erfindung vor, dass die Nabe eines der Messräder mit einer Radmessnabe (Radkraftdynamometer) ausgerüstet ist. Eine solche Ausbildung, wobei auf vorhandene, komplette Radmessnaben zurückgegriffen werden kann, ermöglicht eine kontinuierliche Aufzeichnung der resultierenden Kräfte (Fₓ, F_{y}, F_{z}) sowie der dazugehörigen Momente (Mₓ, M_{y}, M_{z}).

Da je nach Vorschriften auch ein umweltbedingt veränderter Zustand der Straße überprüft werden muss, ist es von Vorteil, wenn die Halterung für die Messräder mit einer Sprüheinrichtung für Wasser und/oder einer Zugabeeinrichtung für Sand, Schmirgel oder Ähnliches ausgerüstet ist. Auf diese Weise ist es möglich, die Straße mit Wasser glitschig zu machen oder andere veränderte Zustände für die Überprüfung vorzugeben.

Was nun die Prognose des Zustandes der Straßendeckschicht betrifft, so wird hierbei ein vorteilhafter Trick insofern angewendet, als die jeweils überprüfte Straßenoberfläche nun noch poliert und noch einmal gemessen wird. Dies wird möglich gemacht, weil gemäß der Erfindung zusätzlich zu den Messrädern eine radial arbeitende Polierbürste dem Rotationsantrieb zugeordnet ist, der in unterschiedliche Rotationsgeschwindigkeiten einstellbar ist. Weiter vorn ist bereits darauf hingewiesen worden, dass Geschwindigkeiten von 40, 60 und 80 km/h für die Messräder erreicht werden sollen, wobei für die Poliereinheit ein noch hochtourigerer Betrieb notwendig ist. 500 U/min. sind notwendig. Wenn ein einziger Rotationsantrieb zum Einsatz kommt, muss er auf die unterschiedlichen Rotationsgeschwindigkeiten eingestellt werden, was gemäß der Ausbildung möglich ist. Damit kann mit einem einzigen Rotationsantrieb gearbeitet werden, über den sowohl die Polierbürste wie auch die Messräder angetrieben werden und zwar jeweils im Wechsel. Ein gleichzeitiger Betrieb ist nicht vorgesehen. Hierdurch ist eine Griffigkeitsprognose möglich, weil über den Rotationsantrieb bzw. die Polierbürsten die noch anstehende Verkehrsbelastung vorteilhaft und sicher simuliert werden kann.

Das Simulierten der Verkehrsbelastung ist insbesondere vorteilhaft möglich, wenn die Polierbürste mit Noppen aus Gummi, deren Härtegrad festgelegt ist, ausgerüstet ist. Damit kann mit dieser Polierbürste das Überfahren des entsprechenden Streckenabschnittes durch LKWs, PKWs und andere Fahrzeuge genau simuliert werden, sodass nach entsprechender Überprüfung gesagt werden kann, welche Belastung der nicht polierte Streckenabschnitt bzw. Streckenbelag noch aushalten kann.

Natürlich werden die Noppen aus Gummi durch den Poliervorgang abgenutzt oder gar zerstört. Dabei ist ein Austausch problemlos möglich, weil die Noppen gruppenweise auswechselbar mit der rotierenden Tragplatte des Gerätegehäuses verbindbar sind. Sie sind beispielsweise dort angeschraubt, sodass sie schnell abgelöst und durch neue ersetzt werden können.

Um den Betrieb mit einem einzigen Rotationsantrieb zu ermöglichen, ist vorgesehen, dass die Polierbürste zwischen den beiden Messrädern angeordnet, aber gegenüber diesen getrennt absenkbar und wieder anhebbar ausgebildet ist. Dadurch ist ein wechselseitiger Betrieb mit ein und demselben Antrieb möglich, wobei der nicht benötigte Geräteteil vom Antrieb zweckmäßigerweise abgekoppelt werden sollte.

Um sicherzustellen, dass jeweils auch nur der Bereich des zu überprüfenden Straßenbelages poliert wird, der für die Messung notwendig ist, sind die Noppen an den Kurzseiten der rechteckigen Tragplatte im Laufbereich der Messräder in einem Halbbogen angeordnet sind. Wird die Tragplatte nun in Rotation versetzt, so entsteht eine polierte, kreisrunde Laufbahn, auf der dann später die Messräder genau geführt und für die Messung eingesetzt werden.

In aller Regel ist es wegen der hohen Umdrehungsgeschwindigkeiten für die Polierbürste zweckmäßig, wenn die Polierbürste über einen eigenen Rotationsantrieb verfügend ausgebildet ist und über einen Schwenkarm um die Mittelachse in die Arbeitsposition im Wechsel mit den Messrädern verschwenkbar ist. Hierbei wird also die Polierbürste mit ihrem Rotationsantrieb in die Arbeitsposition und wieder daraus heraus geschwenkt, sodass eine gegenseitige Beeinflussung von Polierbürste und Messrädern nicht zu befürchten ist. Jeweils nur ein Geräteteil kann in die Arbeitsposition gebracht werden, die dann für das andere Geräteteil gesperrt ist. Bei dieser Ausbildung kann der Rotationsantrieb auf den jeweiligen Bedarf gezielt eingestellt werden, was Vorteile hat und auch kostenmäßig günstiger ist.

Das Gerätegehäuse, das die Messräder, die Polierbürste, die Rotationsantriebe und auch die Messeinrichtung aufnimmt, besteht aus Aluminium, wobei das Gerätegehäuse die Durchführung eines stationären Messverfahrens ermöglichend stabil und gut transportabel ausgebildet und vorzugsweise mit drei Standfüßen und/oder Rollfüßen ausgerüstet ist. Dadurch kann dieses Prüfgerät an jeder beliebigen Stelle beispielsweise auf einem Straßenbelag aufgestellt und dann in Betrieb gesetzt werden, ohne dass die Gefahr besteht, dass sich das Prüfgerät oder Teile desselben verselbstständigen. Außerdem können so insbesondere die schnell rotierenden Polierbürsten in einem geschlossenen Gehäuse betrieben werden, sodass Gefährdungen des Bedienungspersonals nicht bestehen. Ein "Verfahren" von Einsatzort zu Einsatzort ist problemlos über die Rollfüße möglich.

Zur Messung der Griffigkeit von Fahrbahnoberflächen von Straßen und Wegen wird ein Verfahren eingesetzt, das mit dem schräg gestellten Laufrad verwirklicht wird und wobei das Laufrad aus zwei im Abstand angeordneten Messrädern mit Messreifen besteht und wobei die Seitenkraft und die Radlast kontinuierlich und stationär im Labor oder Feld gemessen und die gesamten ermittelten Messdaten ausgewertet werden, wobei die Messräder über einen als Rotationsantrieb ausgelegten Antrieb angetrieben und entlang einer kreisförmigen Bahn über den Antrieb gezogen werden.. Ein solches Verfahren gibt somit die Möglichkeit, sowohl im Labor Prüfungen durchzuführen, wie diese Prüfungen dann auch im Feld zu verwirklichen. Das entsprechende Prüfgerät ist weiter vorne beschrieben worden. Der Einsatz des Gerätes ermöglicht die Verwirklichung des beschriebenen Verfahrens. Damit können genaue Messdaten ermittelt werden, die dann so umgerechnet werden, dass sich daraus die für die Beurteilung der Griffigkeit und auch der Prognose notwendigen Einzelwerte ergeben.

Um von vorne herein mit kritischen Werten arbeiten oder sie gezielt simulieren zu können, ist vorgesehen, dass die Fahrbahnoberfläche mit Wasser vor Durchführung der Messung angenässt und nach der Messung über eine normierte Polierbürste eine vorgegebene Abnutzung stilisierend poliert und an dieser Stelle dann eine erneute stationäre Messung vorgenommen wird. Damit wird verhindert, dass man nur einen trockenen Straßenzustand überprüft, der für die wirkliche effektive Griffigkeit nicht die sicherste Aussage erbringen kann. Dies erfolgt eben wie beschrieben mit einer mit Wasser angenässten Fahrbahnoberfläche. Weiter ist von Vorteil, dass aufgrund der Verwirklichung von stationären Messungen die Möglichkeit besteht, die Messung auch zu wiederholen und so die ermittelten Werte gleich am Ort oder im gewissen Abstand durch weiteres Messen zu überprüfen. Dies hat erhebliche Vorteile bezüglich der Sicherheit der Aussage und ermöglicht es erstmals, konkrete Aussagen über den Zustand im Messzeitpunkt aber auch Prognosen über den Zeitrahmen des Erhaltens eines zulässigen Zustands abgeben zu können. So können dann die Straßenbehörden rechtzeitig oder gar frühzeitig ihre Baumaßnahmen planen und einleiten, sodass eine Gefährdung durch eine Fahrbahnoberfläche mit zu geringer Griffigkeit gar nicht mehr auftreten kann.

Die Messung mit dem schräg gestellten Laufrad auf der vorgefundenen Laufbahn bzw. Fahrbahnoberfläche und der polierten Fahrbahnoberfläche ist besonders aussagekräftig, weil der als reibungs- bzw.
griffigkeitscharakterisierende Größe anzusehende Seitenkraft- oder der Seitenreibungsbeiwert µ_{RAG} als Quotient aus der auf die Messräder wirkenden Normalkraft F_{N} und der reibungsbedingten Kompnente F_{Ry'} der gemessenen Seitenkraft F_{y'} berechnet wird. Die einzelnen Grunddaten können sicher ermittelt werden, sodass dann die entsprechenden Beurteilungskriterien herausgearbeitet und herausgestellt werden können.

Die Erfindung zeichnet sich insbesondere dadurch aus, dass ein vorteilhaft gut zu transportierendes Prüfgerät geschaffen ist, über das die Straßendeckschichten überprüft und auch Prognosen darüber abgegeben werden können. Vorteilhafterweise kann dieses transportable Prüfgerät im Labor, aber eben auch im Straßenbereich eingesetzt werden, wobei bei zweifelhaften Messdaten oder bei sonstigen Problemen ohne großen Aufwand die Messung an gleicher Stelle oder an weiter zurückliegender Stelle noch einmal wiederholt werden kann. Auch wenn dort zur Fertigung der Prognose ein Polierversuch stattgefunden hat, ist es problemlos im Nahbereich der ursprünglichen Stelle möglich, praktisch die gleichen Fahrbahnoberflächendaten noch einmal zu erhalten und so die gesamte Messung zu optimieren bzw. abzusichern. Da das Prüfgerät quasi ein selbst fahrendes Fahrzeug ist, also nicht irgendwie an einen LKW oder ein anderes Zugfahrzeug angehängt werden muss, kann es auch nicht zur Überbelastung der Laufräder bzw. der Messräder kommen, sondern vielmehr kann gezielt an den Stellen gemessen werden, wo eine Beurteilung des Gesamtzustandes der Straßenoberfläche am besten möglich ist. Erstmals kann so beispielsweise auch im Bereich von Rillen eine genaue Messung durchgeführt werden und ermittelt werden, ob trotz des optimalen Zustandes der sonstigen Umgebung nicht doch eine frühzeitige Ausbesserung der Laufbahn bzw. Fahrbahn notwendig ist.

Weitere Einzelheiten und Vorteile des Erfindungsgegenstandes ergeben sich aus der nachfolgenden Beschreibung der zugehörigen Zeichnung, in der ein bevorzugtes Ausführungsbeispiel mit den dazu notwendigen Einzelheiten und Einzelteilen dargestellt ist. Es zeigen:
- Figur 1: ein Prüfgerät mit einem einzigen Rotationsantrieb in perspektivischer Wiedergabe,
- Figur 2: das Prüfgerät nach Figur 1 im Schnitt,
- Figur 3: ein Querschnitt im Bereich der Laufräder, aber oberhalb der Polierbürste,
- Figur 4: die Darstellung der am Messrad wirkenden Kräfte mit schematisiert wiedergegebenen Laufbahnen für die Messräder,
- Figur 5: ein prinzipielle Wiedergabe eines Prüfgerätes mit getrennten Antrieben für das Griffigkeitsmessgerät und das Poliergerät und
- Figur 6: die Wiedergabe der Prinzipskizze nach Figur 5 mit Wiedergabe des in die Arbeitsposition geschwenkten Griffigkeitsmessgerätes.

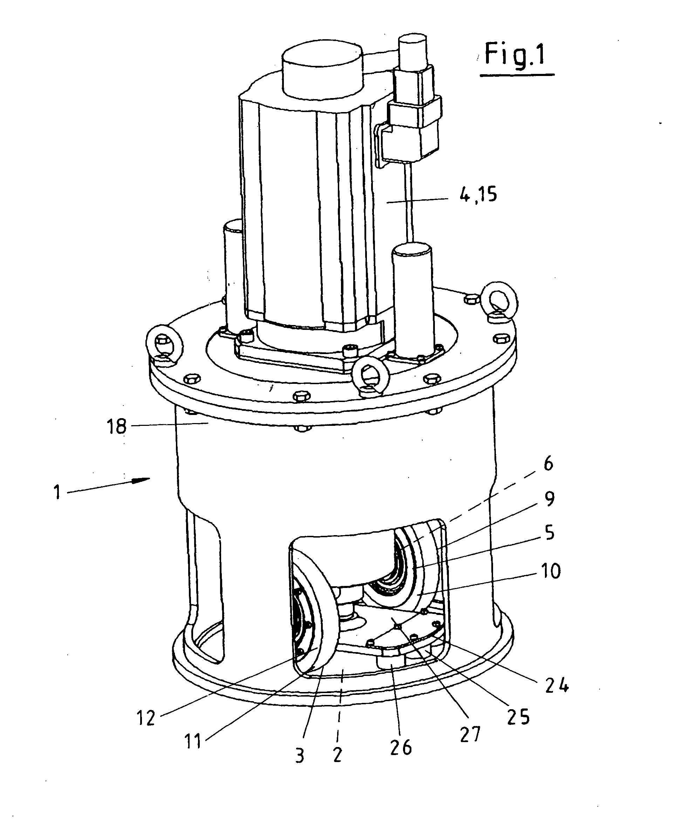

Figur 1 zeigt das Prüfgerät 1, das auf einer Probefläche einer Fahrbahndeckschicht 2 abgestellt ist. Als über den Antrieb 4 angetriebenes Laufrad 5 kommen hier Messräder 9, 11 zum Einsatz, die über einen Rotationsantrieb 15 angetrieben sind und dementsprechend ähnlichen Bedingungen unterliegen, wie das schräg gestellt Laufrad nach dem SCRIM-Verfahren. Durch die bedingte Anlehnung an das SCRIM-Verfahren soll die Vergleichbarkeit der Messwerte mit den flächendeckend vorliegenden SCRIM-Werten bisheriger Überprüfungen ermöglicht werden. Abweichend vom SCRIM-Verfahren ist das hier mit Prüfgerät 1 verwirklichte Verfahren ein stationäres Messverfahren, das sowohl in Labor- wie in Feldversuchen einsetzbar ist. Durch den rotierenden Betrieb der Messräder ist eine wesentlich verbesserte und intensivere Beurteilung der Probefläche möglich.

Die Messreifen 10, 12 der Messräder 9, 11 laufen auf einer kreisförmigen Bahn 3, sodass mit der Messeinrichtung 6 die notwendigen Daten ermittelt werden können. Hierbei sind die Messräder 9, 11 unter 20° schräg gestellt, sodass die darauf wirkende Seitenkraft kontinuierlich gemessen werden kann. Die Messräder 9, 11 rollen vorzugsweise auf einer mit Wasser angenässten Prüfoberfläche oder eben auf der Fahrbahndeckschicht. Der Seitenkraft- oder Seitenreibungswert µ_{RAG} wird dabei als Quotient aus der auf das Messrad 9, 11 wirkenden Normalkraft F_{N} und der reibungsbedingten Komponenten F_{Ry'} der gemessenen Seitenkraft F_{y'} berechnet.

Neben dem Antrieb 4 bzw. Rotationsantrieb 15, den Messrädern 9, 11 und der Messeinrichtung 6 sind dem diese aufnehmenden Gerätegehäuse 18 auch noch eine Polierbürste 24 mit Noppen 25, 26 zugeordnet. Diese Polierbürste 24 ist zwischen den Messrädern 9, 11 positioniert und kann im Wechsel mit diesen über den Rotationsantrieb 15 angetrieben werden. Die Noppen 25, 26 sind lösbar mit einer rotierenden Tragplatte 27 verbunden und zwar in Form eines Halbbogens 29 an den Kurzseiten 28 der rotierenden Tragplatte 27.

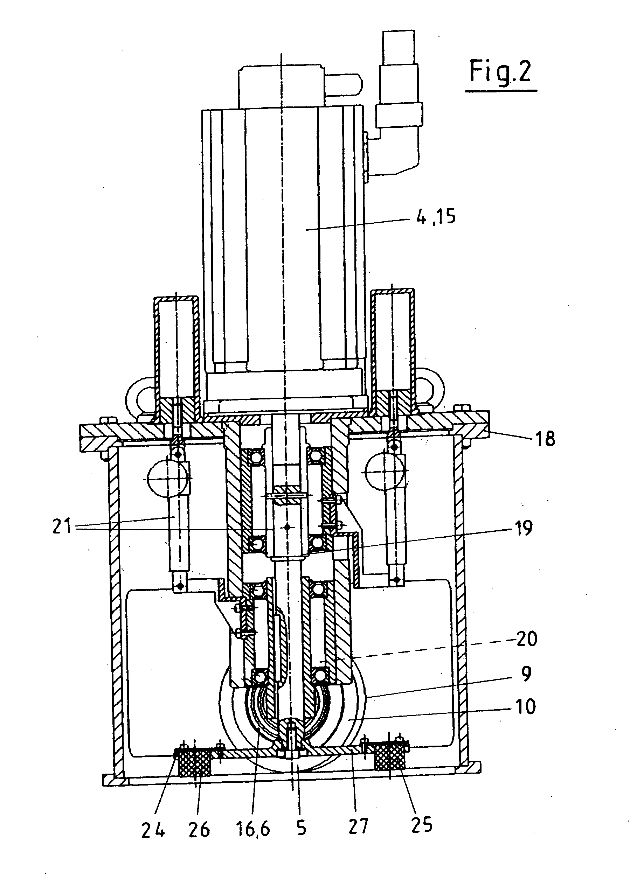

In Figur 2 ist ein Schnitt durch das in Figur 1 gezeigte Prüfgerät 1 wiedergegeben, wobei hier noch die Heb- und Senkvorrichtung 21 erkennbar ist, mit der entweder die Polierbürste 24 oder die Messräder 9, 11 angehoben oder in Position gebracht werden. Erkennbar ist hier das Messrad 9 mit dem Messreifen 10, wobei in die Felge 16 die Messeinrichtung 6 integriert ist.

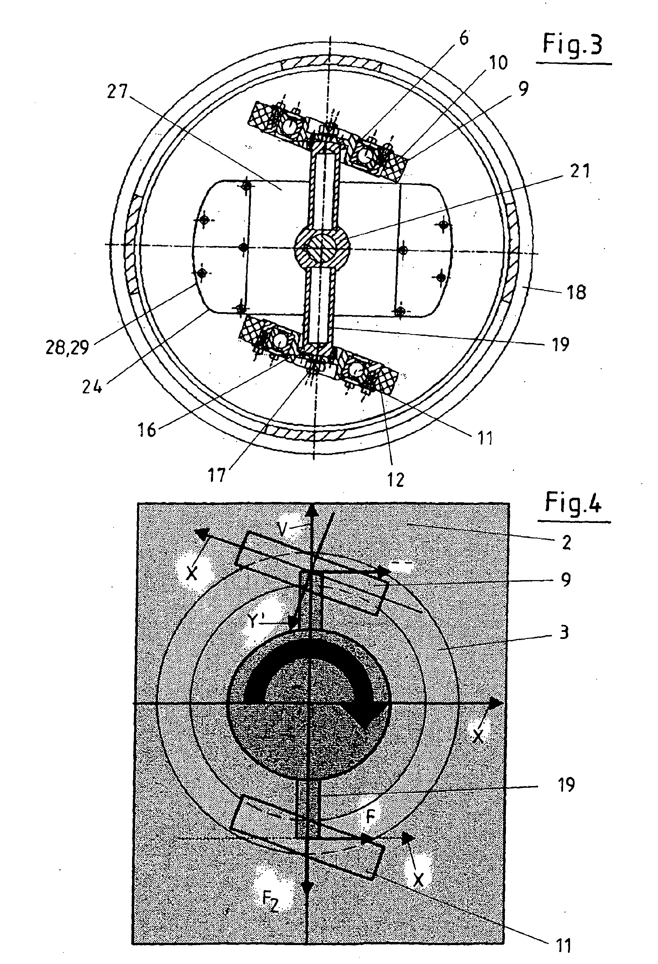

Figur 3 lässt erkennen, dass auch die Nabe 17 zur Aufnahme einer Messeinrichtung dienen kann, wobei zur Messung der auftretenden Seitenkraft und der Radlast bzw. der dadurch auftretenden Kräfte Druckaufnehmer vorgesehen sein können oder eine als Radmessnabe ausgebildete Nabe 17. Erkennbar sind außerdem Kugellager, die es ermöglichen, dass die jeweilige Messeinrichtung 6 im Bereich der Felge 16 untergebracht ist, um möglichst nah die entsprechenden Daten abnehmen zu können. Mit 19 ist die Halterung für die Messräder 9, 11 bezeichnet, während Figur 2 noch eine Sprüheinrichtung 20 entnommen werden kann, über die eine gleichmäßige Benässung der Fahrbahndeckschicht vor bzw. während des Messvorgangs möglich ist.

Figur 4 zeigt eine schematisierte Wiedergabe eines Ausschnittes der Fahrbahndeckschicht 2, die überprüft wird oder werden soll. Mit 3 ist die Bahn bezeichnet, die von den Messrädern 9, 10 "überlaufen" wird und die bei Einsatz der Polierbürste 24 auch noch poliert wird, bevor dann die Messung noch einmal wiederholt wird oder auch in mehreren Stufen. Diese Messräder 9, 11 werden über Halterungen 19 in Position gehalten, sodass sie die vorgegebene Schrägstellung immer einhalten, auch wenn sie wie weiter vorne schon mehrfach beschrieben, während der Rotation bemüht sind, in Laufrichtung zu schwenken. Darüber ist es dann möglich, die entsprechenden Messdaten abzugreifen, wobei hier in Figur 4 insbesondere die Darstellung der auf das Messrad 9 bzw. 11 wirkenden Reibungskraft F_{R} und der in axialer Richtung wirkenden Zentrifugalkraft F_{Z} wiedergegeben werden soll.

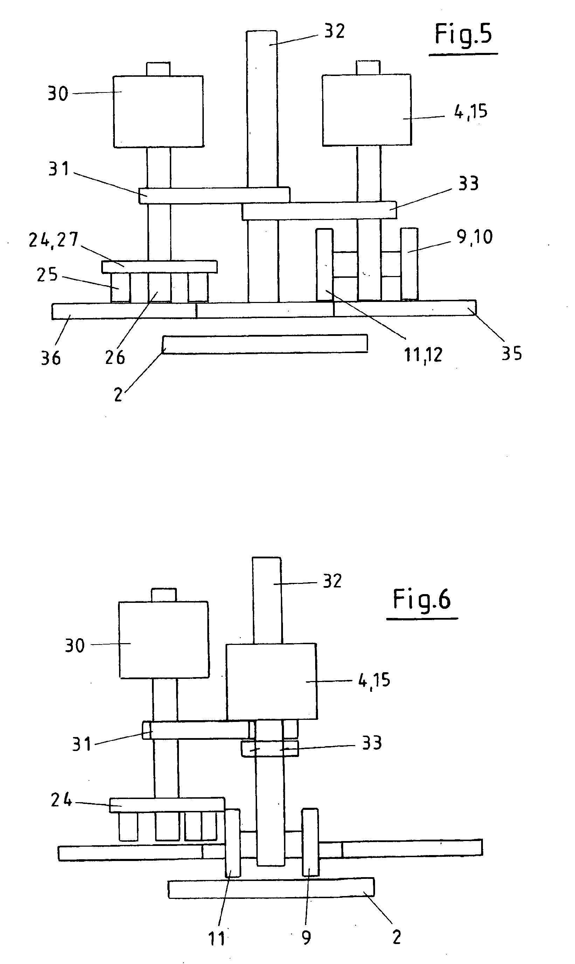

Die Figuren 5 und 6 schließlich zeigen ein Prüfgerät 1, bei dem die Messräder 9, 11 einen Rotationsantrieb 15 und die Polierbürste 24 einen Rotationsantrieb 30 aufweist. Hier haben beide Einheiten jeweils einen eigenen Rotationsantrieb, sodass insbesondere die hohen Drehwerte für die Polierbürste 24 problemlos erbracht werden können, ohne auf die anderen Bauteile negativ einzuwirken. Um entweder die Polierbürste 24 oder die Messräder 9, 11 in Arbeitsposition zu bringen, sind Schwenkarme 31 bzw. 33 vorgesehen, die um die Mittelachse 32 schwenken. Nach Figur 6 ist dieser Schwenkvorgang dann für die Messräder 9, 11 vollzogen, sodass diese sich in der Frontposition befinden und nun auf der Probefläche bzw. der Fahrbahndeckschicht 2 arbeiten können. Leicht erkennbar ist, dass genau so auch nach dem Zurückschwenken des Rotationsantriebes 15 für die Messräder 9, 11 in die aus Figur 5 ersichtliche Position nun der Rotationsantrieb 30 mit der Polierbürste 24 in Arbeitsposition gebracht und abgesenkt werden kann.

Die Messräder 9, 11 rollen für die Messung der Griffigkeit auf der ebenen, kreisförmigen Prüfoberfläche 2 mit einem Durchmesser von ca. 225 mm. Als Messgeschwindigkeiten können 40, 60 und 80 km/h gewählt werden. Beim Einsatz der Polierbürste 24 werden über den zugeordneten Rotationsantrieb 500 U/min. und ggf. mehr verwirklicht.

## Patentansprüche

1. Prüfgerät zur Messung der Griffigkeit von Fahrbahndeckschichten (2) mit einem in einem bestimmten Winkel entlang einer Bahn (3), schräg laufenden Laufrad (5) und einer die notwendigen Messdaten ermittelnden Messeinrichtung wobei das Laufrad (5) rotierend und zwei beabstandete, schräg laufende Messräder (9, 11) mit Messreifen (10, 12) aufweisend ausgebildet ist, dass und wobei die Messeinrichtung (6) den Messrädern (9, 11) zugeordnet und die Seitenkraft und die Radlast oder Normalkraft ermittelnd ausgeführt ist,
**dadurch gekennzeichnet,**
**dass** die Messräder (9, 11) mit einem als Rotationsantrieb (15) ausgelegten Antrieb (4) ausgerüstet sind und entlang einer kreisförmigen Bahn (3) über den Antrieb (4) gezogen sind.

2. Prüfgerät nach Anspruch 1,
**dadurch gekennzeichnet,**
**dass** die Messeinrichtung (6) einer der Felgen (16) des Messreifens (10) zugeordnet ist.

3. Prüfgerät nach Anspruch 1,
**dadurch gekennzeichnet,**
**dass** das rotierende Laufrad (5) mit den Messrädern (9, 11) die durch den Rotationsantrieb (15) und die durch die Messreifen (10, 12) entstehenden Schwingungen entkoppelnd im Gerätegehäuse (18) gelagert ist.

4. Prüfgerät nach einem der vorhergehenden Ansprüche,
**dadurch gekennzeichnet,**
**dass** der Seitenkraft- oder Seitenreibungsbeiwert µ_{RAG} als Quotient aus der auf das rotierende Laufrad (5) wirkenden Normalkraft F_{N} und der reibungsbedingten Komponente F_{Ry'} der gemessenen Seitenkraft F_{y'} ermittelbar ist, wobei die gemessene Seitenkraft F_{y'} sich aus der Summe der in Richtung y' wirkenden Komponenten der Zentrifugalkraft F_{Zy'} und Reibungskraft F_{Ry'} ergibt.

5. Prüfgerät nach Anspruch 2,
**dadurch gekennzeichnet,**
**dass** die einer der Felgen (16) zugeordnete Messeinrichtung (6) ein Kraftaufnahmesystem zur Bestimmung der Seitenkraft F_{y'} und der Radlast F_{N} aufweist.

6. Prüfgerät nach einem der vorhergehenden Ansprüche,
**dadurch gekennzeichnet,**
**dass** die Messeinrichtung (6) zur Messung der auftretenden Seitenkraft und der Radlast bzw. der dadurch auftretenden Kräfte Druckaufnehmer aufweist.

7. Prüfgerät nach einem der vorhergehenden Ansprüche,
**dadurch gekennzeichnet,**
**dass** die Messeinrichtung (6) zur Messung der Seitenkraft zusätzlich oder nur Dehnungsmessstreifen (DMS) aufweist, die zu einer Vollbrücke verschaltet sind.

8. Prüfgerät nach einem der vorhergehenden Ansprüche,
**dadurch gekennzeichnet,**
**dass** die Nabe (17) eines der Messräder (9) mit einer Radmessnabe (Radkraftdynamometer) ausgerüstet ist.

9. Prüfgerät nach einem der vorhergehenden Ansprüche,
**dadurch gekennzeichnet,**
**dass** die Halterung (19) für die Messräder (9, 11) mit einer Sprüheinrichtung (20) für Wasser und/oder einer Zugabeeinrichtung für Sand, Schmirgel oder Ähnliches ausgerüstet ist.

10. Prüfgerät nach einem der vorhergehenden Ansprüche,
**dadurch gekennzeichnet,**
**dass** zusätzlich zu den Messrädern (9, 11) eine radial arbeitende Polierbürste (24) dem Rotationsantrieb (15) zugeordnet ist, der in unterschiedliche Rotationsgeschwindigkeiten einstellbar ist.

11. Prüfgerät nach Anspruch 10,
**dadurch gekennzeichnet,**
**dass** die Polierbürste (24) mit Noppen (25, 26) aus Gummi, deren Härtegrad festgelegt ist, ausgerüstet ist.

12. Prüfgerät nach einem der vorhergehenden Ansprüche,
**dadurch gekennzeichnet,**
**dass** die Polierbürste (24) zwischen den beiden Messrädern (9, 11) angeordnet, aber gegenüber diesen getrennt absenkbar und wieder anhebbar ausgebildet ist.

13. Prüfgerät nach einem der vorhergehenden Ansprüche,
**dadurch gekennzeichnet,**
**dass** die Polierbürste (24) über einen eigenen Rotationsantrieb (30) verfügend ausgebildet ist und über einen Schwenkarm (31) um die Mittelachse (32) in die Arbeitsposition im Wechsel mit den Messrädern (9, 11) verschwenkbar ist.

14. Verfahren zur Messung der Griffigkeit von Fahrbahnoberflächen von Straßen und Wegen, wobei über ein bei etwa 10 - 20° gegenüber der Geraden schräg gestelltes Laufrad neben anderen notwendigen Messdaten die Kraft ermittelt wird, mit der das Laufrad versucht sich in die Fahrtrichtung zu verschwenken, wobei das Laufrad aus zwei im Abstand angeordneten Messrädern mit Messreifen besteht, und wobei die Seitenkraft und die Radlast kontinuierlich und stationär im Labor oder Feld gemessen und die gesamten ermittelten Messdaten ausgewertet werden,
**dadurch gekennzeichnet,**
**dass** die Messräder über einen als Rotationsantrieb ausgelegten Antrieb angetrieben und entlang einer kreisförmigen Bahn über den Antrieb gezogen werden.

15. Verfahren nach Anspruch 14,
**dadurch gekennzeichnet,**
**dass** die Fahrbahnoberfläche mit Wasser vor Durchführung der Messung angenässt und nach der Messung über eine normierte Polierbürste eine vorgegebene Abnutzung stilisierend poliert und an dieser Stelle dann eine erneute stationäre Messung vorgenommen wird.

## Claims

1. Test equipment for measuring the skid resistance of road surface layers (2) with a running wheel (5) running obliquely at a specific angle along a track (3) and a measuring instrument (6) that measures the necessary measured data, whereby the running wheel (5) is formed to rotate and to have two spaced, obliquely running measuring wheels (9, 11) with measuring tyres (10, 12), that and whereby the measuring instrument (6) is assigned to the measuring wheels (9, 11) and designed to measure the lateral force and the wheel load or normal force,
**characterised in that**
the measuring wheels (9, 11) are equipped with a drive (4) designed as a rotary drive (15) and are drawn by the drive (4) along a circular track (3).

2. Test equipment in accordance with Claim 1,
**characterised in that**
the measuring instrument (6) is placed on one of the rims (16) of the measuring tyres (10).

3. Test equipment in accordance with Claim 1,
**characterised in that**
that the rotating running wheel (5) is on bearings on the appliance housing (18) with the measuring wheels (9, 11) so that oscillations caused by the rotary drive (15) and the measuring tyres (10, 12) are decoupled.

4. Test equipment in accordance with one of the above Claims,
**characterised in that**
the lateral force or lateral friction coefficient µ_{RAG} can be measured as the quotient of the normal force F_{N} impacting the rotating running wheel (5) and the frictional component F_{Ry} of the measured lateral force F_{y}, whereby the measured lateral force F_{y'} results from the sum of the components of centrifugal force F_{Zy'} and frictional force F_{Ry'} acting in direction y'.

5. Test equipment in accordance with Claim 2,
**characterised in that**
the measuring instrument (6) allocated to one of the rims (16) has a force absorption system for measuring the lateral force F_{y'} and the wheel load F_{N}.

6. Test equipment in accordance with one of the above Claims,
**characterised in that**
the measuring instrument (6) for measuring the occurring lateral force and the wheel load or the forces arising from this has a pressure sensor.

7. Test equipment in accordance with one of the above Claims,
**characterised in that**
the measuring instrument (6) for measuring the lateral force has additional or only strain gauges (DMS) that are interconnected to a full bridge.

8. Test equipment in accordance with one of the above Claims,
**characterised in that**
the hub (17) of one of the measuring wheels (9) is fitted with a wheel measuring hub (wheel force dynamometer).

9. Test equipment in accordance with one of the above Claims,
**characterised in that**
the bracket (19) for the measuring wheels (9, 11) is fitted with a spray device (20) for water and/or a dispensing device for sand, abrasive or similar.

10. Test equipment in accordance with one of the above Claims,
**characterised in that**
in addition to the measuring wheels (9, 11) a radially working polishing brush (24) is assigned to the rotary drive (15) that can be set at different rotation speeds.

11. Test equipment in accordance with Claim 10,
**characterised in that**
the polishing brush (24) is equipped with nubs (25, 26) made of rubber whose degree of hardness is stipulated.

12. Test equipment in accordance with one of the above Claims,
**characterised in that**
the polishing brush (24) is located between the two measuring wheels (9, 11), but is designed to be separately lowerable and elevatable again with regard to them.

13. Test equipment in accordance with one of the above Claims,
**characterised in that**
the polishing brush (24) is designed to have its own rotary drive (30) and can be swivelled via a swivel arm (31) around the centre axis (32) into the working position alternately with the measuring wheels (9, 11).

14. Procedure for measuring the skid resistance of the surfaces of roads and paths, whereby along with other necessary measured data the force with which the running wheel attempts to swivel into the direction of running is measured using a running wheel at an angle of 10°-20° to the straight line, whereby the running wheel consists of two spaced measuring wheels with measuring tyres, and whereby the lateral force and the wheel load are measured continuously and stationarily in the laboratory or the field and the complete measured data are evaluated,
**characterised in that**
the measuring wheels are driven by a drive designed as a rotary drive and are drawn by the drive along a circular track.

15. Procedure in accordance with Claim 14,
**characterised in that**
the road surface is wetted with water before measuring is carried out and after measuring a given level of wear is polished stylising using a standardised polishing brush and renewed stationary measuring is then carried out at this position.

## Revendications

1. Appareil de test destiné à mesurer l'adhérence des couches de roulement de chaussée (2) avec une roue de roulement (5) roulant en biais avec un certain angle le long d'une chaussée (3), et un dispositif de mesure (6) relevant les données de mesure nécessaires, sachant que la roue de roulement (5) est conçue rotative et présentant deux roues de mesure (9, 11) roulant en biais, écartées l'une par rapport à l'autre, avec des pneus de mesure (10, 12), sachant que le dispositif de mesure (6) est attribué aux roues de mesure (9, 11) et est conçu de manière à déterminer la force latérale et la charge de roue, ou la force normale,
**caractérisé par le fait**
**que** les roues de mesure (9, 11) sont équipées d'un entraînement (4) conçu en tant qu'entraînement rotatif (15) et sont tirées par l'entraînement (4) le long d'une chaussée (3) en forme de cercle.

2. Appareil de test selon la revendication 1,
**caractérisé par le fait**
**que** le dispositif de mesure (6) est attribué à une des jantes (16) du pneu de mesure (10).

3. Appareil de test selon la revendication 1,
**caractérisé par le fait**
**que** la roue de roulement rotative (5) est amortie dans le boîtier de l'appareil (18) par les roues de mesure (9, 11), de façon à être découplée des vibrations qui apparaissent sous l'effet de l'entraînement rotatif (15) et du pneu de mesure (10, 12).

4. Appareil de test selon une des revendications précédentes,
**caractérisé par le fait**
**que** le coefficient de la force latérale ou de frottement transversal µ_{RAG} peut être déterminé, en tant que quotient, à partir de la force normale F_{N} agissant sur la roue de roulement rotative (5) et de la composante dépendant du frottement F_{Ry'} de la force latérale mesurée F_{y'}, sachant que la force latérale F_{y'} résulte de la somme des composants agissant dans le sens y' de la force centrifuge F_{Zy'} et de la force de frottement F_{Ry'}.

5. Appareil de test selon la revendication 2,
**caractérisé par le fait**
**que** le dispositif de mesure (6) attribué à une des jantes (16) présente un système de réception de force destiné à déterminer la force latérale F_{y'} et la charge de roue F_{N}.

6. Appareil de test selon une des revendications précédentes,
**caractérisé par le fait**
**que** le dispositif de mesure (6) destiné à mesurer la force latérale et la charge de roue resp. les forces qui apparaissent de ce fait présente un capteur de pression.

7. Appareil de test selon une des revendications précédentes,
**caractérisé par le fait**
**que** le dispositif de mesure (6) présente, pour mesurer la force latérale, en sus ou bien uniquement des jauges de contrainte qui sont commutées de façon à former un pont complet.

8. Appareil de test selon une des revendications précédentes,
**caractérisé par le fait**
**que** le moyeu (17) d'une des roues de mesure (9) est équipé d'un moyeu de mesure de roue (dynamomètre de la force de la roue).

9. Appareil de test selon une des revendications précédentes,
**caractérisé par le fait**
**que** le support (19) des roues de mesure (9, 11) est équipé d'un dispositif de pulvérisation (20) de l'eau et/ou un dispositif d'adjonction pour le sable, l'émeri ou autre matériau semblable.

10. Appareil de test selon une des revendications précédentes,
**caractérisé par le fait**
**qu'**une brosse de polissage fonctionnant dans le sens radial (24) est attribuée, en sus des roues de mesure (9, 11), à l'entraînement rotatif (15) qui peut être réglé à différentes vitesses de rotation.

11. Appareil de test selon la revendication 10,
**caractérisé par le fait**
**que** la brosse de polissage (24) est équipée de picots (25, 26) en caoutchouc, dont le degré de dureté est défini.

12. Appareil de test selon une des revendications précédentes,
**caractérisé par le fait**
**que** la brosse de polissage (24) est disposée entre les deux roues de mesure (9, 11), mais conçue pour pouvoir être abaissée et à nouveau soulevée séparément de ces dernières.

13. Appareil de test selon une des revendications précédentes,
**caractérisé par le fait**
**que** la brosse de polissage (24) est conçue en disposant de son propre entraînement de rotation (30) et pour pouvoir pivoter au moyen d'un bras pivotant (31) autour de l'axe médian (32) dans la position de travail en alternance avec les roues de mesure (9, 11).

14. Procédé destiné à mesurer l'adhérence de surfaces de chaussées de rues et de chemins, sachant que la force avec laquelle la roue de roulement tend à basculer dans le sens de la marche est déterminée par l'intermédiaire d'une roue de roulement placée en biais avec environ 10 - 20° par rapport aux lignes droites, outre d'autres données de mesure nécessaires, sachant que la roue de roulement consiste en deux roues de mesure disposées écartées munies de pneus de mesure, et sachant que la force latérale et la charge de roue et l'ensemble des données de mesure enregistrées sont évaluées de façon continue et stationnaire en laboratoire ou sur le terrain,
**caractérisé par le fait**
**que** les roues de mesure sont tirées par un entraînement conçu sous la forme d'un entraînement rotatif et le long d'une chaussée en forme de cercle.

15. Procédé selon la revendication 14,
**caractérisé par le fait**
**que** la surface de la chaussée est, avant l'exécution de la mesure, mouillée avec de l'eau et est, après la mesure, polie au moyen d'une brosse de polissage normée de façon à styliser une usure prescrite, et qu'une nouvelle mesure stationnaire est alors effectuée à cet endroit.
